# EUROPEAN PATENT APPLICATION

(11) **EP 1 532 976 A2**
(43) Date of publication of application: **25.05.2005**
(21) Application number: 04257119.0
(22) Date of filing: 17.11.2004
(51) Int. Cl.: A61K 31/045, A61K 31/075, A61K 31/095, A61K 31/10, A61K 31/195, A61K 31/198, A61K 38/06, A61K 38/28, A61K 41/00, A61P 35/00

(54) **Compositions comprising organic disulfides, cysteine sulfoxides, sulfones, sulfoximines or other sulfur containing substances and their use in therapy in combination with radiation**

(30) Priority: 18.11.2003 GB 0326870; 07.04.2004 GB 0407993; 16.08.2004 GB 0418363
(71) Applicant: Holt, John Alfred Gorton, Perth, Western Australia 6009 (AU); Holt, Mark Rowan Gorton, Horsham, West Sussex RH12 2GB (GB)
(72) Inventor: Holt, John Alfred Gorton, Nedlands, WA 6009 Perth (AU)
(74) Representative: Brown, David Leslie

(57) **Abstract**

The present invention provides novel therapies for treating a range of conditions, including cancer, enhancing T-cell count, or reducing the concentration of pathogenic particles (e.g. virus or prion particles) - most especially those selected from human immunodeficiency virus (HIV), bovine spongiform encephalopathy (BSE) agent, scrapie agent, hepatitis agent, disseminated ("multiple") sclerosis agent, Creutzfeld Jacob disease (CJD) agent, variant CJD agent, systemic lupus erythematosis (SLE) agent, ankylosing spondylitis agent and amyotrophic sclerosis agent futhermore amyloid conditions, Alzheimer's disease, and non-malignant diseases - applicable *in vivo* to a patient in need of such treatment, the therapies comprising administering to the patient an effective amount of defined active agents, and, while the agents are present, administering to the patient an effective dose of electromagnetic radiation of frequency in the range of about 400-450 MHz.

## Description

### Field of the Invention

The present invention relates to therapeutic methods and compositions for use therein.

### Background of the Invention

European Patent Application No. 0531031 (the disclosure of which is incorporated herein by reference) describes a cancer therapy, and compositions for use therein, in which an effective amount of a non-toxic agent selected from organic disulfides, oxidising agents and organic sulfoximines is administered to a patient and while said agent is present at a cancer site of the patient there is administered to the cancer site an effective dose of electromagnetic radiation of frequency in the range of about 400-450 MHz.

Penicillamine disulfide (3,3,3',3'-tetramethylcystine), which has the formula 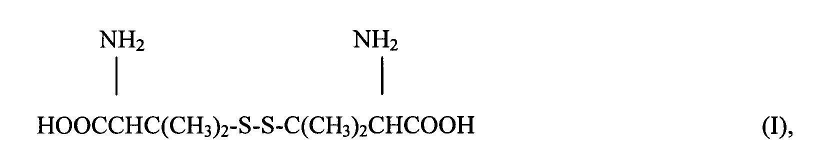 or a non-toxic salt thereof, is mentioned as a suitable organic disulfide. A particularly preferred disulfide is stated to be cystine, which has the formula 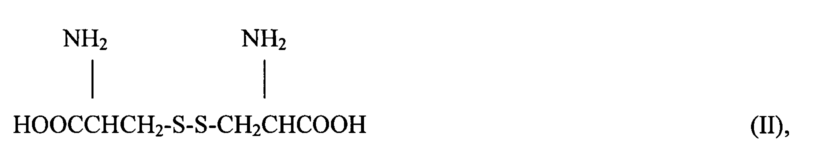 or a non-toxic salt thereof.

Methionine sulfoximine, which has the formula 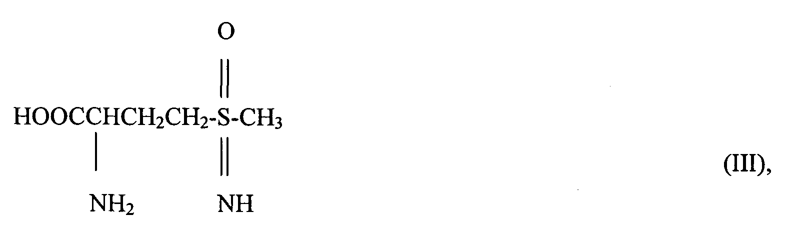 or a non-toxic salt thereof, is mentioned as a suitable organic sulfoximine.

Preferred oxidising agents are stated to be any of the hydroperoxides of cumene and t-butyl hydroperoxide.

The description of EP-A-0531031 states (clarification added in square brackets) "in a preferred form the invention may be said to provide a method of human cancer therapy which comprises administering to a human patient an effective amount of one or more non-toxic compound of general formula [R-S-S-R¹], [R³-O-O-R⁴], [R⁵-O-O-H] or [R⁶-S(O)(NR⁸)-R⁷] ... and while said compound(s) is or are present at a cancer site of the patient administering to the cancer site an effective dose of ... electromagnetic radiation of frequency in the range about 400-450 MHz".

European Patent Application No. 0616803 (the disclosure of which is incorporated herein by reference) describes the use of selected agents from the disclosure of EP-A-0531031 in enhancing blood T-cell count in immunodeficient (e.g. HIV-positive) individuals, the agents being used in conjunction with the electromagnetic radiation substantially as described in EP-A-0531031.

The selected agents for use in the enhancement of blood T-cell count are non-toxic organic disulfides of general formula

R-S-S-R (IV)

in which R is an ethyl group β,β-disubstituted by two substituents selected from amino, carboxy, carboxyl-(C₁₋₄-alkyl)-carbamoyl, C₂₋₅-alkanamido substituted by amino and carboxy, and salt derivatives thereof. The preferred active agent is stated to be L-cystine. Another disulfide agent mentioned is oxidised glutathione, which has the formula 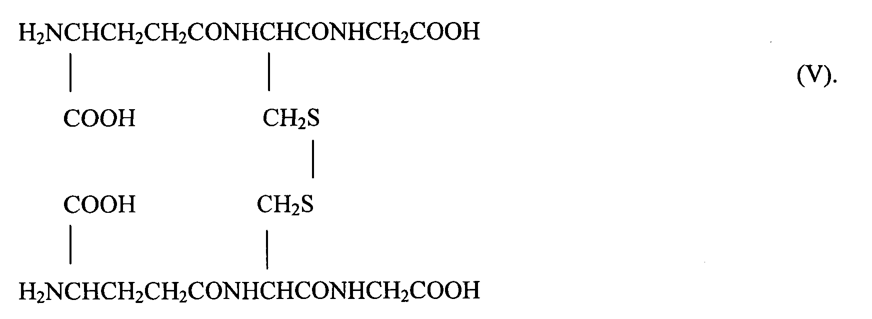 It will be noted that the formula IV covers cystine and oxidised glutathione, but excludes penicillamine disulfide.

European Patent Application No. 0705603 (the disclosure of which is incorporated herein by reference) describes the use of a non-toxic cysteine sulfoxide of general formula 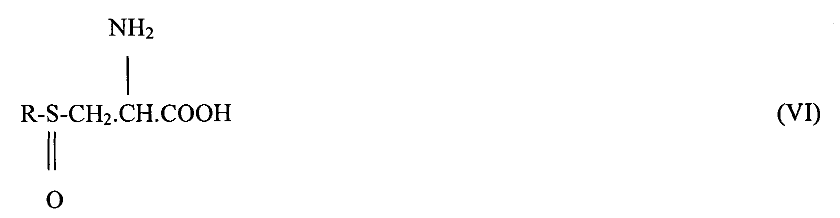 or a non toxic salt thereof, in which R is a C₁₋₄ alkyl or a C₂₋₄ alkenyl group, in conjunction with the electromagnetic radiation substantially as described in EP-A-0531031, to provide a therapeutic method (e.g. an anticancer therapy or a method for enhancing T-cell count) applicable *in vivo* to a patient or *in vitro* to a transfusable body fluid or transplantable body part.

European Patent Application No. 0764442 (the disclosure of which is incorporated herein by reference) describes the use of cystine, oxidised glutathione, the cysteine sulfoxides of general formula VI and the non-toxic salts thereof, and the electromagnetic radiation, for reducing the concentration of pathogenic viral and/or prion particles in body tissue and fluids.

### Brief Description of the Invention

The present invention is based on my surprising finding that the efficacy of the prior art methods can potentially be enhanced if the active agents include (a) a non-toxic organic disulfide selected from the group consisting of penicillamine disulfide (I) or a non-toxic salt thereof, a compound of formula IV or a non-toxic salt thereof (e.g. cystine, oxidised glutathione or a non-toxic salt thereof), and a disulfide-bridged protein or a non-toxic salt thereof, and (b) a non-toxic cysteine sulfoxide of general formula VI or a non-toxic salt thereof, or a compound of general formula 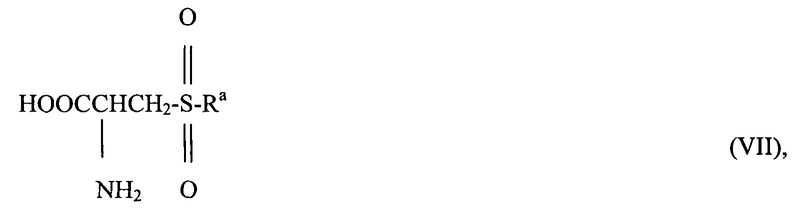 or a non-toxic salt thereof, in which R^{a} is H, OH or a C₁₋₄ alkyl or a C₂₋₄ alkenyl group.

Furthermore, I have discovered that the efficacy of the treatment may be substantially preserved or in some cases even enhanced, if the compound of formula VII or a non-toxic salt thereof is used in the therapy without the component (a) or the other option for component (b) necessarily being present.

Furthermore, I have discovered that compounds of general formula 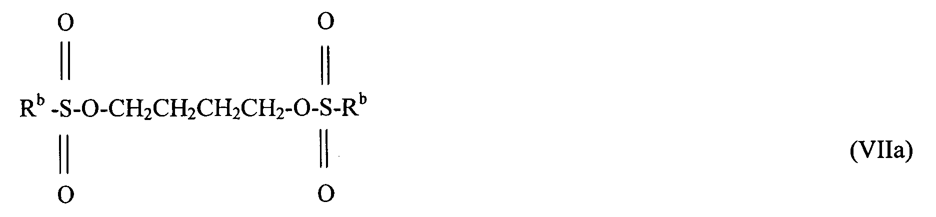 in which R^{b} is a C₁₋₄ alkyl or a C₂₋₄ alkenyl group, preferably methyl, can be used in the treatment when administered in the form of an oral composition, without the component (a) or (b) necessarily being present.

According to a first aspect of the present invention, there is provided the use, in the following therapeutic method or in the manufacture of one or more compositions for use in the following therapeutic method, of:
(a) one or more non-toxic organic disulfide selected from the group consisting of penicillamine disulfide (I) or a non-toxic salt thereof, a compound of formula IV or a non-toxic salt thereof (e.g. cystine, oxidised glutathione or a non-toxic salt thereof), and a disulfide-bridged protein or a non-toxic salt thereof, and
(b) one or more non-toxic cysteine sulfoxide of general formula VI or a non-toxic salt thereof, one or more compound of general formula VII or a non-toxic salt thereof, or any combination thereof; the said therapeutic method being selected from treating cancer, enhancing T-cell count, or reducing the concentration of pathogenic particles (e.g. virus or prion particles) - most especially those selected from human immunodeficiency virus (HIV), bovine spongiform encephalopathy (BSE) agent, scrapie agent, hepatitis agent, disseminated ("multiple") sclerosis agent, Creutzfeld Jacob disease (CJD) agent, variant CJD agent, systemic lupus erythematosis (SLE) agent, ankylosing spondylitis agent and amyotrophic sclerosis agent ― applicable *in vivo* to a patient in need of such treatment, the method comprising administering to the patient an effective amount of the agents (a) and (b), and, while the agents are present, administering to the patient an effective dose of electromagnetic radiation of frequency in the range of about 400-450 MHz.

According to a second aspect of the present invention, there is provided the use, in the following therapeutic method or in the manufacture of a composition for use in the following therapeutic method, of:
(a) one or more non-toxic organic disulfide selected from the group consisting of penicillamine disulfide (I) or a non-toxic salt thereof, a compound of formula IV or a non-toxic salt thereof (e.g. cystine, oxidised glutathione or a non-toxic salt thereof), and a disulfide-bridged protein or a non-toxic salt thereof;
(b) one or more non-toxic cysteine sulfoxide of general formula VI or a non-toxic salt thereof, one or more compound of general formula VII or a non-toxic salt thereof, or any combination thereof; and
(c) one or more organic sulfoximine of general formula 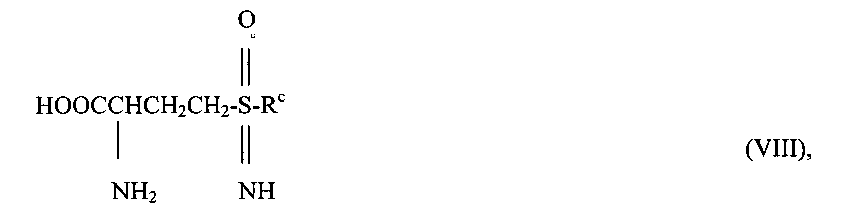 or a non-toxic salt thereof, wherein R^{c} is a C₁₋₄ alkyl group, for example methyl or butyl;

the said therapeutic method being selected from treating amyloid conditions, Alzheimer's disease, and non-malignant diseases such as SLE, multiple sclerosis and scleroderma, applicable *in vivo* to a patient in need of such treatment, the method comprising administering to the patient an effective amount of the agents (a), (b) and (c), and, while the agents are present, administering to the patient an effective dose of electromagnetic radiation of frequency in the range of about 400-450 MHz.

According to a third aspect of the present invention, there is provided a method of treating cancer, of enhancing T-cell count, or of treating acquired immunodeficiency syndrome (AIDS), bovine spongiform encephalopathy (BSE), scrapie, hepatitis, disseminated ("multiple") sclerosis, Creutzfeld Jacob disease (CJD), variant CJD, systemic lupus erythematosis (SLE), ankylosing spondylitis and amyotrophic sclerosis *in vivo* in a patient in need of such treatment, the method comprising administering to the patient an effective amount of the agents (a) and (b) as defined above in the first aspect of the present invention, and, while the agents are present in the patient administering to the patient an effective dose of electromagnetic radiation of frequency in the range of about 400-450 MHz.

According to a fourth aspect of the present invention, there is provided a method of treating amyloid conditions, Alzheimer's disease, and non-malignant diseases such as SLE, multiple sclerosis and scleroderma *in vivo* in a patient in need of such treatment, the method comprising administering to the patient an effective amount of the agents (a), (b) and (c) as defined above in the second aspect of the present invention, and, while the agents are present in the patient administering to the patient an effective dose of electromagnetic radiation of frequency in the range of about 400-450 MHz.

According to a fifth aspect of the present invention, there is provided a kit for use in a method as defined in the first or third aspect of the present invention, the kit comprising a first composition or compositions containing the (a)-agent or agents and a second composition or compositions containing the (b)-agent or agents, optionally together with instructional material for the use.

According to a sixth aspect of the present invention, there is provided a kit for use in a method as defined in the second or fourth aspect of the present invention, the kit comprising a first composition or compositions containing the (a)-agent or agents, a second composition or compositions containing the (b)-agent or agents, and a third composition or compositions containing the (c)-agent or agents, optionally together with instructional material for the use.

The following combinations of agents (a) and (b) are excluded from the uses, methods and kits of the first, third and fifth aspects of the invention:
for treatment of cancer, the combination of (a) cystine, oxidised glutathione, any non-toxic salt of either, or any mixture or combination thereof and (b) one or more non-toxic cysteine sulfoxide of general formula VI, any non-toxic salt thereof, or any mixture or combination thereof (which are disclosed for that use in EP-A-0705603); and
for reducing the concentration of pathogenic particles, the combination of (a) cystine, oxidised glutathione, any non-toxic salt of either, or any mixture or combination thereof and (b) one or more non-toxic cysteine sulfoxide of general formula VI, any non-toxic salt thereof, or any mixture or combination thereof (which are disclosed for that use in EP-A-0764442).

In one particular embodiment of the invention, the active agents consist essentially of the agents (a), (b) and - if present - (c) as defined above.

According to a further aspect of the present invention, there is provided an aqueous pharmaceutical composition comprising (a) one or more non-toxic organic disulfide selected from the group consisting of penicillamine disulfide (I) or a non-toxic salt thereof, a compound of formula IV or a non-toxic salt thereof (e.g. cystine, oxidised glutathione or a non-toxic salt thereof), and a disulfide-bridged protein or a non-toxic salt thereof, and (b) one or more non-toxic cysteine sulfoxide of general formula VI or a non-toxic salt thereof, one or more compound of general formula VII or a non-toxic salt thereof, or any combination thereof; with the proviso that when the composition includes the specific combination of (a) cystine, oxidised glutathione, any non-toxic salt of either, or any mixture or combination thereof and (b) one or more non-toxic cysteine sulfoxide of general formula VI, any non-toxic salt thereof, or any mixture or combination thereof, then at least one further compound from those defined for (a) and (b) in the first aspect of the present invention (e.g. one or more of penicillamine disulfide, a compound of general formula VII or a non-toxic salt thereof) is also present.

This aqueous pharmaceutical composition is useful as one of the admininstered compositions in a preferred aspect of the method according to the present invention.

According to an eighth aspect of the present invention, there is provided the use, in the following therapeutic method or in the manufacture of one or more compositions for use in the following therapeutic method, of one or more compound of general formula VII or a non-toxic salt thereof, one or more compound of general formula VIIa, or any combination thereof; the said therapeutic method being selected from treating cancer, enhancing T-cell count, treating amyloid conditions, Alzheimer's disease, and non-malignant diseases such as SLE, multiple sclerosis and scleroderma or reducing the concentration of pathogenic particles (e.g. virus or prion particles) ― most especially those selected from human immunodeficiency virus (HIV), bovine spongiform encephalopathy (BSE) agent, scrapie agent, hepatitis agent, disseminated ("multiple") sclerosis agent, Creutzfeld Jacob disease (CJD) agent, variant CJD agent, systemic lupus erythematosis (SLE) agent, ankylosing spondylitis agent and amyotrophic sclerosis agent - applicable *in vivo* to a patient in need of such treatment, the method comprising administering to the patient an effective amount of the one or more compound of general formula VII or a non-toxic salt thereof, one or more compound of general formula VIIa, or any combination thereof, and, while the compound or compounds are present, administering to the patient an effective dose of electromagnetic radiation of frequency in the range of about 400-450 MHz.

According to a ninth aspect of the present invention, there is provided a method of treating cancer, of enhancing T-cell count, of treating amyloid conditions, Alzheimer's disease, and non-malignant diseases such as SLE, multiple sclerosis and scleroderma or of treating acquired immunodeficiency syndrome (AIDS), bovine spongiform encephalopathy (BSE), scrapie, hepatitis, disseminated ("multiple") sclerosis, Creutzfeld Jacob disease (CJD), variant CJD, systemic lupus erythematosis (SLE), ankylosing spondylitis and amyotrophic sclerosis *in vivo* in a patient in need of such treatment, the method comprising administering to the patient an effective amount of one or more compound of general formula VII or a non-toxic salt thereof, one or more compound of general formula VIIa, or any combination thereof, and, while the said compound or compounds are present in the patient administering to the patient an effective dose of electromagnetic radiation of frequency in the range of about 400-450 MHz.

If desired, the one or more compound of general formula VII or a non-toxic salt thereof, one or more compound of general formula VIIa, or any combination thereof, can be administered in association with one or more of the other components (a), (b) and (c) as defined above.

According to a tenth aspect of the present invention, there is provided a kit for use in a method as defined in the eighth or the ninth aspect of the present invention, the kit comprising a composition containing one or more compound of general formula VII or a non-toxic salt thereof, one or more compound of general formula VIIa, or any combination thereof, optionally in association with one or more additional composition or compositions containing one or more of the other the (a)-agent or agents, (b)-agent or agents, and (c)-agent or agents as defined above, optionally together with instructional material for the use.

According to a further aspect of the present invention, there is provided an aqueous pharmaceutical composition comprising one or more compound of general formula VII or a non-toxic salt thereof, one or more compound of general formula VIIa, or any combination thereof, and a co-agent selected from (a) one or more non-toxic organic disulfide selected from the group consisting of penicillamine disulfide (I) or a non-toxic salt thereof, a compound of formula IV or a non-toxic salt thereof (e.g. cystine, oxidised glutathione or a non-toxic salt thereof), and a disulfide-bridged protein or a non-toxic salt thereof, (b) one or more non-toxic cysteine sulfoxide of general formula VI or a non-toxic salt thereof, or (c) one or more one or more organic sulfoximine of general formula (VIII) or a non-toxic salt thereof; or any combination thereof.

This aqueous pharmaceutical composition is useful as one of the admininstered compositions in a preferred aspect of the method according to the present invention.

The compositions for use in the methods of the present invention preferably consist essentially of the stated active agent or combination of agents with a pharmaceutically acceptable carrier, diluent, excipient or combination thereof.

Where the compound of general formula VII is cysteic acid, it is preferably not administered intravenously. Intravenous administration of cysteic acid can cause vein thromboses. This risk can, however, be reduced in the intravenous administration route by using the cysteic acid in small doses or, more preferably, in combination with other active agents as stated above.

Where the compound of general formula VIIa in which R^{b} is methyl is used in accordance with the invention, it is preferably formulated in a composition for oral administration and administered orally.

The present invention is primarily intended for treatment of human patients, both adults and children.

### Detailed Description of the Invention

### The Active Agents

The expression "salt" used herein includes acid and base addition salts, for example salts of strong acids, e.g. hydrochloric or sulphuric acid, or strong bases, e.g. sodium hydroxide or potassium hydroxide.

The alkyl groups R in general formula VI, R^{a} in general formula VII, R^{b} in general formula VIIa and the group R^{c} in general formula VIII may suitably be selected from methyl, ethyl, n-propyl, s-propyl, n-butyl, s-butyl and t-butyl, and preferably n-butyl.

An optically active form of penicillamine disulfide, or alternatively an optically inactive (internally compensated) form or a racemic mixture, may be used. The dextrorotatory (D) form is preferred. The non-toxic salts are preferably non-toxic acid or base addition salts, for example addition salts of strong acids or bases. A preferred active agent of formula I is D-penicillamine disulfide.

The compound of general formula VI for use in the present invention is preferably S-methyl cysteine sulfoxide or a non-toxic salt thereof. An optically active form of the cysteine sulfoxide of general formula VI, or alternatively a racemic mixture, may be used. The laevorotatory (L) form is preferred. The non-toxic salts are preferably non-toxic acid or base addition salts, for example addition salts of strong acids or bases. A preferred sulfoxide active agent is S-methyl-L-cysteine sulfoxide.

The compound of general formula VII for use in the present invention is preferably S-methyl cysteine sulfone, α-cysteine sulfonic acid, cysteic acid or a non-toxic salt of any of these compounds. An optically active form of the compound of general formula VII, or alternatively a racemic mixture, may be used. The laevorotatory (L) form is preferred. The non-toxic salts are preferably non-toxic acid or base addition salts, for example addition salts of strong acids or bases.

The compound of general formula VIIa for use in the present invention is preferably busulfan (R^{b} = methyl), 1,4-butanediol dimethane sulfonate ester.

The compound of general formula VIII for use in the present invention is preferably buthionine sulfoximine or a non-toxic salt thereof. An optically active form of buthionine sulfoximine, or alternatively a racemic mixture, may be used. The DL-buthionine-DL-sulfoximine form is preferred. Methionine sulfoximine, particularly the DL-methionine-DL-sulfoximine form, is an alternative example of the compound of general formula VIII, but is less preferred.

The disulfide-bridged protein may be any protein or polypeptide in which regions of a chain or different chains are linked by one or more disulfide or disulfide-containing bridges. Examples of such proteins include insulin, e.g. mammalian insulin, whether from human, animal or synthetic (recombinant) sources.

In mammalian insulin, for example, three disulfide bridges are present. Mammalian insulin consists of two polypeptide chains, an A chain composed of 21 amino acids and a B chain composed of 30 amino acids. The two chains being linked by two disulfide bridges (A7 to B7 and A20 to B19) and the A chain having a disulfide bridge between the two regions A6 and A11.

Mixed bacterial vaccines (MBVs) ― also known as Coley's toxins - are vaccines derived from more than one bacterium, typically *Streptococcus* and *Serratia marcasens.* The vaccine mixture may typically contain bacterial toxins (e.g. endotoxins) and/or dead and/or attenuated bacterial cells. See, for example Kolmel *et al,* Onkologie 1991, 14, pp 411-417; Zhao *et al,* Med. Oncol. & Tumor Pharmacother. 1991, 8, pp 23-28; Johnston *et al,* Cancer Chemotherapy Reports, August 1962, 21, pp19-68; Nauts, Prog. Clin. Biol. Res. 1982, 107, pp 687-696. Recent research on MBVs has shown that they include disulfide-bridged proteins and it is believed that they are suitable for use in the present invention. Therefore, in a further example the disulfide-bridged protein may be used in the form of an MBV composition comprising the protein.

Included within the scope of the invention is the use of non-toxic prodrugs or precursors of the said agents, which are administered to the patient or the body fluid or body part and are modified *in situ* by reaction to provide the desired agent at the therapeutic site. For example, a thiol can be administered simultaneously with a non-toxic oxidising agent (for example one or more organic hydroperoxide such as cumene hydroperoxide, t-butyl hydroperoxide, or a combination thereof) to provide the corresponding disulfide (usually in the presence of some unreacted thiol) and optionally an excess of the oxidising agent. A compound of general formula VI can be administered simultaneously with a non-toxic oxidising agent (for example one or more organic hydroperoxide such as cumene hydroperoxide, t-butyl hydroperoxide, or a combination thereof) to provide the corresponding compound of general formula VII (usually in the presence of some unreacted compound of general formula VI) and optionally an excess of the oxidising agent.

A non-toxic oxidising agent, for example one or more organic hydroperoxide such as cumene hydroperoxide, t-butyl hydroperoxide, or a combination thereof, may be administered with the agents to generally prevent or restrict degradation of those agents, e.g. reduction of the agents *in vivo,* particularly in the liver. The agents will typically be present in the administered composition(s) with the oxidising agent, suitably in an aqueous saline solution.

The above definitions of the agents to be used in the present invention are intended to include within their scope functionally similar derivatives and other forms thereof, as will be readily appreciated by those skilled in this art.

### The Compositions and Administration Protocols

The aqueous composition form(s) by which the agents are administered may take any suitable form and employ any suitable conventional pharmaceutical carrier(s) or excipient(s).

Apart from insulin all the agents should be administered parenterally for the *in vivo* methods. The composition form is preferably an aqueous solution or suspension suitable for intravenous or percutaneous drip, infusion or injection. Intravenous drip, infusion or injection is the preferred administration route by virtue of its rapid delivery of the active agents to the bloodstream or to a cancer site with minimal degradation of the active agents. In some patients, irritation of the vein may occur, or no large veins may be available, in which case percutaneous infusion using a percutaneous infusion central catheter (PICC) may be used.

The agents may be provided in one composition or in more than one composition. They are preferably presented as aqueous formulations, optionally including one or more suitable inactive excipient, as is known in pharmacology.

The agents may be administered simultaneously or in any desired order or sequence. The method may, for example, include administering the compositions provided for by the kit, in which the (a)-agent or agents are present in one or more compositions separately from the (b)-agent or agents, and the (c)-agent or agents, when present, provided in one or more compositions again.

The disulfide cystine (II) must be used with a solubilising or suspending agent. The solubilising or suspending agent, when present, is preferably N-methyl-glucamine, which has the formula

CH₃.NH.CH₂.CH(OH).CH(OH).CH(OH).CH(OH).CH₂OH (IX).

An optically active form of N-methyl-glucamine, or alternatively an optically inactive (internally compensated) form or a racemic mixture, may be used. The dextrorotatory (D) form is preferred. A preferred solubilising agent is N-methyl-D-glucamine.

All the other disulfides for use in the present invention ― e.g. penicillamine disulfide, oxidised glutathione and non-toxic salts thereof - are water-soluble.

The expression "non-toxic" used herein refers to an acceptably low level of toxicity when a compound is present at an effective amount, and not necessarily to a complete absence of toxic effects. In particular, compounds which have only a temporary toxic effect and do no significant permanent harm, or for which any toxic effects can readily be counteracted by the administration of a non-toxic "antidote" agent, will be referred to and understood as "non-toxic" herein. The expression "non-toxic" also refers to the usage of the active agents in non-toxic but effective amounts according to the present invention. Penicillamine disulfide, the cysteine sulfoxides of general formula VI, the compounds of general formula VII, and their salts, are generally non-toxic in doses exceeding 100 mg per kilogram body weight. Oxidised glutathione can generally be used in effective amounts according to the present invention without toxic effects. The toxicity data for the agents to be used in the present invention is either readily obtainable from published sources, or the determination of the maximum effective non-toxic dosages of the agents to be used in the present invention is within the capacity of one of ordinary skill in this art without undue effort.

The compounds of general formula VIII, for example buthionine sulfoximine or methionine sulfoximine, must be used on a daily basis at a dosage of less than 0.2 gm/kg in females and less than 0.5 gm/kg in males. There is a possibility of hypoglycaemic (low glucose levels) symptoms on the brain at higher dosages.

Where the agents are to be administered simultaneously, this may be done by including the agents in one composition or in more than one, simultaneously administered, composition. The more than one composition may include the same or a different combination of agents and other components as each other, and where an agent is present in more than one of the compositions then that agent can be in the same or in different concentrations as between the different compositions.

Where the agents are to be administered sequentially, this may be done by dividing a single composition into more than one portion for administration, or by providing more than one, sequentially administered, composition. The more than one composition may include the same or a different combination of agents and other components as each other, and where an agent is present in more than one of the compositions then that agent can be in the same or in different concentrations as between the different compositions.

The aqueous composition may if desired include additional components such as salts (e.g., sodium chloride), further solubilising and/or suspending agents and the like. Such additional components will generally be selected from known components for pharmaceutical compositions, as will be well understood by those skilled in this art. As mentioned above, non-toxic (physiologically compatible) oxidants such as organic hydroperoxides, for example cumene hydroperoxide and/or t-butyl hydroperoxide, may also be present.

The compositions comprising the parts of the kit will preferably be provided in suitable containers (e.g. bags suitable for percutaneous or intravenous delivery), which may, if desired, be packaged together for transportation and storage, and may be accompanied by instructional material describing the therapeutic method. Alternatively, the compositions and/or the kit may be prepared in a clinic or the like, immediately before use.

The compositions are prepared by standard mixing techniques that will be readily apparent to those skilled in this art.

The composition(s) for intravenous *in vivo* use are generally prepared to deliver an effective amount of the active agents to the patient over an administration period in the range of up to about 15 minutes, suitably up to about 2 to 5 minutes, at an intravenous administration rate of up to approximately 20 ml per minute. For example, a 25 ml dose of a first composition may be administered intravenously or via PICC line and immediately followed by a 25 ml dose of a second composition administered intravenously or via PICC line, over a total influx time period of two to three minutes.

### The Diseases

The expression "cancer" used herein refers to all forms of neoplasia and precursors thereof. There may particularly be mentioned a malignant neoplasm of the epithelium (for example, a carcinoma such as squamous cell carcinoma, basal cell carcinoma, transitional cell carcinoma, adenocarcinoma or cystadenocarcinoma), pleural and/or peritoneal surfaces (for example, mesothelioma of those surfaces), pigment cells (for example, a malignant melanoma), neural or brain tissue (for example, neurofibrosarcoma, malignant glioma, medulloblastoma or neuroblastoma), kidney tissue (for example, a nephroblastoma), eye tissue (for example, a retinoblastoma), connective tissue (for example, a sarcoma or meningioma such as myxosarcoma, fibrosarcoma, liposarcoma, Kaposi's sarcoma, chondrosarcoma, osteosarcoma, myosarcoma, leiomyosarcoma, rhabdomyo-sarcoma, angiosarcoma, haemangiosarcoma, lymphangiosarcoma or malignant meningioma), lymphoreticular and haematopoietic tissue (for example, a malignant non-Hodgkin's lymphoma, lymphocytic leukaemia, plasmacytoma and multiple myeloma, reticulum cell sarcoma, Hodgkin's disease, granulocytic leukaemia or monocytic leukaemia) or embryonic-originating tissue (for example, a malignant teratoma, teratocarcinoma or chorioncarcinoma). The method of the present invention may also be used to treat benign neoplasia.

The expression "pathogenic particles" used herein includes disease-causing infective particular agents having a size smaller than bacterial pathogens, which are growing exponentially *in vivo* or in culture media (*in vitro*). Examples of such particles are viruses and prions. Prion particles are generally smaller than viral particles. Virus- and prion-propagated diseases treatable by the method of the invention include, principally, acquired immunodeficiency syndrome (AIDS), bovine spongiform encephalopathy (BSE or "Mad Cow" disease), scrapie, hepatitis, disseminated ("multiple") sclerosis (MS), Creutzfeld Jacob disease (CJD), variant CJD. systemic lupus erythematosis (SLE), ankylosing spondylitis and amyotrophic lateral sclerosis. The corresponding viral and/or prion particles are destroyed when growing exponentially, by the method of the invention.

The expression "amyloid diseases" used herein includes, for example, extra-cranial malignant amyloidosis, amyloid polyneuropathy, transthyretin (TTR) amyloid disease and senile systemic amyloidosis (SSA).

The therapeutic method made available by the present invention can be used in both human and veterinary (domestic and commercial) medicine. Human treatments are a preferred aspect of the present invention.

### Administration of the Electromagnetic Radiation

After administration of the active agents according to the invention, the electromagnetic radiation should normally be begun to be administered between about 1 and 5 minutes later.

To administer the electromagnetic radiation *in vivo,* the patient is brought close to a suitable number of microwave antennae (e.g. 3 or 4 antennae arranged to encircle the patient's body) and the antennae energised to transmit the electromagnetic radiation. Normally the patient will be moved under the antennae.

The total antennae power should preferably be less than about 4 kW (e.g. adjustable between about 1 and about 3kW) in order to remain tolerable to the average patient. Patients may preferably be treated in two or more sessions, preferably three sessions, per day at daily intervals or every other or every third day for a treatment period of up to about four weeks.

Preferably, each session involves intravenous administration of the active agents in the compositions described above followed over the subsequent 30 minutes by from 1 to about 5 exposures (e.g. three exposures) to the electromagnetic radiation, each exposure to the radiation lasting for about 5 to 10 minutes, with a 2 to 5 minute rest period between exposures. The total length of treatment on each session day will be dependent on the length of time over which the agents remain in the patient's body without substantial loss, and the length of rests between exposures which the patient requires. For optimum results each session should be terminated within 30 minutes of the first agent being administered. Small bodies (e.g. children) and adults can normally tolerate the same radiation power of about 1.6 to 2 kW per session.

As mentioned above, the electromagnetic radiation should be in the frequency range of about 400-450 MHz. More preferably, the frequency should be in the range of about 425-450 MHz, most preferably 432-436 MHz (e.g. about 434 MHz).

Each antenna may consist of a single circular turn approximately 19 cm in diameter (the diameter being chosen in order that it resonates at the frequency of the transmitter to create minimum reflected power), the plane of the circular antenna being held substantially tangential to the curvature of the patient's body, and within about 10 cm from the patient's skin. Such a circular antenna may emit predominantly H-wave electromagnetic radiation of wavelength between about 65 and 75 cm (most preferably about 69 cm) towards the patient's body.

While the presence of E-wave radiation generated by dipole antennae (folded or of other configuration) will not necessarily hinder the efficacy of the radiation, it can lead to greater heating of the patient's skin and a poorer depth dose than a circular or loop antenna. Such effects are undesirable and dipole antennae are therefore less preferred.

Each antenna is suitably connected to a UHF generator by a coaxial cable. Dipole antennae require circulators to prevent adverse interaction between antennae. Circular or loop antennae, which can be tuned accurately to resonate at the frequency of the generator, do not require circulators. Each generator may if desired be set at a slightly different frequency at or around a central frequency in the range already mentioned (preferably about 434 MHz). Each antenna is preferably energised at about 500 to about 800 W.

All equipment within about 2 metres of the antennae should be of non-metallic materials such as wood or plastic, including for example the patient support table. The antennae themselves should preferably be housed in plastic enclosures, e.g. of polypropylene, with about 35 cm clearance between adjacent enclosures.

If a localised treatment, e.g. of a defined cancer site, is required, the electromagnetic radiation may be directed to that site alone. Alternatively, the whole of the patient's body, or head, neck and torso, may be exposed to the electromagnetic radiation. Whole body exposure is clearly necessary where systemic diseases such as viral and prion infections or leukaemias are concerned, and whole body or head/neck/torso exposure may desirable in other cases, e.g. because of the need to treat possible undetected metastases of cancer or other undetected disease sites. It is generally preferred that the antennae are moved relative to the patient, preferably by fixing the antennae around a movable support for the patient, the antennae typically defining a channel between them through which the patient can pass, lying on the movable support. Four antennae are suitable used, directed to the two lateral sides, and the anterior and posterior sides of the patient.

Administration of the electromagnetic radiation in the *in vitro* method of the invention may be performed by any appropriate electromagnetic radiation emitting device, as will be readily understood by a worker of ordinary skill in this art, provided that steps are taken to prevent overheating of the material being treated.

Tests have yielded the following data, which are included purely for ease of understanding of the present invention, and not for limitation of the scope of the invention:

### EXAMPLE

### Compositions and administration volumes

Three standard solutions for intravenous injections were prepared in three bags, together with a phial of human rapid-acting insulin solution.

**Bag 1:** To one litre of normal (0.9%) saline, add 2.5 to 3.0 ml (i.e. about 2.8 ml, the precise volume within the state range being not critical although not less than this amount) of a 70% solution of t-butyl hydroperoxide in water, 50.0 g of S-methyl-L-cysteine sulfoxide and 1.0 ml of an 80% solution of cumene hydroperoxide. If S-methyl-L-cysteine sulfone is available, replace 25.0 g of S-methyl-L-cysteine sulfoxide with 25.0 g of the sulfone. This bag is made up 3 to 7 days ahead of use, and stored for that period at room temperature (not below 10-15 deg C or above 40 deg C). This storage is believed to convert approximately 50% of the sulfoxide into the sulfone.

**Bag 2:** To one litre of normal (0.9%) saline add 2.5 to 3.0 ml (i.e. about 2.8 ml, the precise volume within the stated range being not critical although not less than this amount) of a 70% solution of t-butyl hydroperoxide in water, 50.0 g of S-methyl-L-cysteine sulfoxide, and 1.0 ml of an 80% solution, in water, of cumene hydroperoxide. 6 g of D-pencillamine disulfide, 30 g of oxidised glutathione, 10 g of cysteic acid and 20 g of cystine. The cystine will create a white precipitate and this solution is cleared by the addition of between 100 and 150 g ofN-methyl-D-glucamine (or more if needed) to render the solution transparent.

**Bag 3:** DL-Methionine sulfoximine (1.0 g dissolved in 1 litre of normal saline) is used as a third composition to treat Alzheimer's disease and extra-cranial malignant amyloidosis. Limits of dosage for females is 0.2 mg/kg of body weight; for males 0.5 mg/kg of body weight.

**Phial 4:** Human Insulin Solution, 100 International Units per 1 ml.

### Preferred Therapy

A subcutaneous injection of 10 international units (I.U.) of Insulin, is given followed 10 minutes later by 25ml of each of Bags 1 and 2, injected directly intravenously or via a PICC line using hand pressure with a total influx time of approximately two to three minutes. This is then followed by three exposures to 434 MHz electromagnetic radiation (ultra high frequency radiowaves) between six and seven minutes each to the appropriate part(s) of the body using a moving couch method at which the patient is travelling at two metres per minute through four antennae each energised to 600 W at frequencies between 433.00 and 435.00 MHz. The four antennae are circular in shape, have accurately adjusted physical dimensions to resonate with the minimum reflected power when connected to each of their respective generators and spaced right and left sided laterally, and anteriorly and posteriorly with a 35 cm clearance between the protective polypropylene boxes in which they are housed.

Some sweating and increased pulse rate and pulse pressure occurs in some patients, which settles after 15 minutes rest in an air-conditioned room with a suitable fan system whilst 500 ml or so of a flavoured glucose drink is given orally.

This therapy is repeated daily as often as is necessary. Treatment is typically given for about 15 days (e.g. Mondays to Fridays over three successive weeks), and then an evaluation of the response is typically made about 6 to 8 weeks later, before any subsequent treatment.

Bag 3 is used additionally to the above regime when treating Alzheimer's disease, amyloid disease, and most non-malignant diseases, e.g. Lupus, Multiple Sclerosis, scleroderma etc., which progress exponentially in severity.

To avoid any side effects (similar to an overdose of insulin in a diabetic person) the patient's diet must contain the amino acid methionine. The consumption of 100 grams of cooked red meat twice weekly avoids side effects from the treatment.

### Toxicity

Approximately 1 %-2% of patients may experience mild disorientation (similar to minor overdosage of insulin in a diabetic condition) which disappears rapidly with an intake (oral) of 10-20 g of glucose. This is caused by the insulin injection, which is reduced by 2 or 3 I.U. for the next day's treatment.

Where the compound of general formula VII is cysteic acid, it is preferably not administered intravenously. Intravenous administration of cysteic acid can cause vein thromboses. This risk can, however, be reduced in the intravenous administration route by using the cysteic acid in small doses and in combination with other active agents.

Where the compound of general formula VIIa in which R^{b} is methyl is used in accordance with the invention, it is preferably formulated in a composition for oral administration and administered orally.

No symptoms suggestive of any other toxicity have been noted after the administration. No immediate or late changes in blood pressure, renal or liver function, electrolytes or haematology have been seen from the method.

### Contra-indications

A prime contraindication is the presence of large collections of fluid in the pleural or peritoneal cavities. Static collections of fluid which contain high concentrations of salts have heat generated in them by the application of these electromagnetic fields. Patients with pleural or peritoneal effusions have blockage of the lymphatics draining these areas and also involvement of the pleural and peritoneal membranes. Unless these cavities are dry (less than 100 ml fluid when treated) the heating effect aggravates the irritation of the pleural and peritoneal cavities and the effusions become worse. Recommendations for treatment of such patients are as follows:
A: Peritoneal effusions. A Le Veen shunt or equivalent should be inserted prior to treatment and must be working so that the peritoneal cavity is kept dry. This drains the ascitic fluid back into the circulation.
B: Pleural effusions. A similar shunt can be used but is much less satisfactory. Pleural paracentesis to drain the cavity together with introduction of some agent causing sclerosis of the pleural cavity which obliterates it is more effective. The introduction of talc, tetracycline or another sclerosing agent appears the best way of preventing failure in the treatment.

Another contraindication to this treatment is where cancer of the stomach or bowel is causing partial obstruction to the gut. Treatment can cause breakdown of some of the cancer with a perforation of the bowel above the obstruction. All patients with bowel cancer should be fully assessed by a surgeon and if there is any evidence of obstruction the primary cancer should be removed or exteriorised by colostomy or some other similar procedure. This avoids the likelihood of a perforation, which can be lethal.

The following Case Histories are presented to show, without limitation, clinical applications of the method of the invention. In some cases, the insulin was omitted from the therapy, and the contents of the (1) and (2) bags were varied within the defined limits of the invention.

### CASE HISTORY 1

PH (male): Diagnosed HIV at age 42 years. T4 level two years later was 300 per cubic millimetre (normal is greater than 700). Treated two months later, when the presence of increasing pulmonary infections indicated he had entered the phase of active acquired immunodeficiency syndrome (AIDS). Treatment was according to the invention. T4 count varying between 500 and 700 seven months later. Retreated three times according to the invention, each over a 15 day period. T4 level recovered to 1250 (normal). His T4-T8 ratio returned to normal. His thrombocytopenia of 60,000 per cubic millimetre returned to 150,000, which is within normal limits (150,000 to 500,000). All his other haematological signs became normal. His secondary thrush infection of the throat disappeared. He no longer took antibiotics. He was tested with the 24VLP antigen and had a normal antigen response to all tests. He was free of detectable HIV virus since two months after said three retreatments, is well with normal haematology and zero level HIV DNA/RNA is detectable in July 2003, 10 years after this first treatment.

### CASE HISTORY 2

LB (female): Diagnosis and History: Adenosquamous cell carcinoma of the mouth together with secondaries in both sides of the neck, treated by surgery at age 53 years before referral to me. First seen by me at age 54 years and 7 months, with a recurrence in the floor of the mouth to the left of the midline, a secondary lymph node in the left neck and recurrence in the scar on the right in the submandibular triangle. All three sites confirmed recurrent malignancy. Treated immediately according to the invention over 18 days with daily treatments. Nine years and ten months later she has no evidence of recurrence of the carcinoma of the floor of the mouth. Two and a half years ago she developed a new primary cancer in her right breast. Biopsy confirmed this to be second primary and she was retreated twice according to the invention. No evidence of activity from either cancer is detectable clinically and all laboratory findings are normal 30 months latter.

### CASE HISTORY 3

GB (female): Diagnosis and History: Recurrent carcinoma of the left breast. A carcinoma of the left breast was treated by excision (partial mastectomy) and she was first seen by me at age 51 years with a 4 cm diameter recurrence above the nipple. No nodes were palpable. Biopsy proved recurrent duct carcinoma of the left breast. Received immediate treatment according to the invention over 16 days with daily treatments, and reduction of the mass to half size subsequently occurred. Retreated according to the invention 3, 30, 57 and 61 months later. 24 months after this final treatment all her cancer antigens were measured and found to be within normal limits. Her CA 15-3 was 16.5 kU/IL (reference 30.0) and her ultrasound and mammography appeared within normal limits. She was last examined 10 years after her original therapy and no cancer was detected.

### CASE HISTORY 4

GC (female): Diagnosis and History: A lumpectomy and axillary clearance for a proven carcinoma of the right breast was performed at age 75 years before referral to me. First seen by me one month later, with an infiltrating duct carcinoma present in the right breast with two secondaries in the right axilla. Course of treatment according to the invention given about 6 weeks later. She was regularly followed up without clinical evidence of activity of her disease at least 5 years and 2 months after the treatment according to the invention. No adjuvant therapy was given.

### CASE HISTORY 5

CF (female): Diagnosis and History: 17 years ago diagnosed with scleroderma which advanced despite conventional treatment. Developed left sided breast cancer and surgery was considered too dangerous because her scleroderma prevented any accurate fine muscle movements in shoulders, arms, forearms and fingers together with oral and oesophageal and gastric insufficiency due to involvement of these organs with scleroderma. First seen 8 years ago and treated according to the invention with a 19 day course. Her breast cancer resolved completely and a very severe reaction (oesophagitis, gastritis, pharyngitis and skin rash) from her scleroderma required 2 months to resolve. A further 19 day course was given 3 years ago for her residual scleroderma and this has been asymptomatic with complete recovery of limb and finger functions. Her breast cancer is undetectable. She has not required any medications for her scleroderma for 3 years.

### CASE HISTORY 6

EJ (female): Diagnosis and History: Squamous cell carcinoma of the floor of the mouth, which was removed, together with four incisor teeth, before referral to me. Submental lymph node was involved and bilateral excision of submental and submandibular lymph nodes was performed before referral to me. She refused further surgery. First seen by me at age 75 years and 8 months, with a recurrence in the floor of the mouth 3 x 4 cm in size, proven to be malignant on biopsy. Two courses of treatment were given immediately according to the invention, the first course lasting for 18 days of daily treatments, then a rest period of 38 days, and the second course lasting for another 18 days of daily treatments. Three years after the last treatment, there was no evidence of disease.

### CASE HISTORY 7

AK (male): Diagnosis and History: A sufferer of tuberculosis in the left upper lung. Biopsy confirmed a small cell carcinoma in the right lower lobe with secondary spread to the nodes in the right hilum of the chest. Patient declined chemotherapy. Radiotherapy not considered. The cancer was inoperable. First seen by me at age 53 years and 11 months, and a 15 day course of treatment according to the invention was given two months after referral. Eights years and six months later the patient was well, with no clinical radiological evidence of the disease.

### CASE HISTORY 8

DL (female): Diagnosis and History: Carcinoma of the breast present for two and a half years when first seen by me at age 50 years and 10 months. She has taken hormones for 18 months and was referred to me when these failed to control the disease. Biopsy of a mass 3 cm in diameter in the right breast proved well differentiated adenocarcinoma. Immediate treatment according to the invention was undertaken. Treatment lasted 18 days at daily intervals. At an examination eight years and three months after the end of the treatment according to the invention, no evidence of malignancy was present.

### CASE HISTORY 9

EM (female): Diagnosis and History: At age about 23 years, her left neck node showed Hodgkin's disease by biopsy together with an enlarged thymus in the chest. Two years of chemotherapy, active recurrence, and residual disease was still present and was treated with massive cytotoxic dosage followed by a stem cell transfusion. This was ineffective, and three further courses of chemotherapy were delivered until reviewed four years later. On referral to me at age 29 years, the patient was given four courses of treatment according to the invention. An examination was conducted one year after the last course. The patient was well, without evidence of disease. Her sedimentation rate had fallen to a normal level and she appears to have controlled the disease, which had previously been completely out of control using conventional therapeutic methods.

### CASE HISTORY 10

WM (female): Diagnosis and History: First seen by me at age 46 years and 5 months, with a six month history of a slowly increasing swelling in the right popliteal fossa, and biopsy proved this to be a leiomyosarcoma. Her knee joint was seriously damaged and following two courses of treatment according to the invention - immediately on first referral and two months later ― a knee reconstruction was essential. This has been satisfactorily performed and the specimen revealed no evidence of malignancy. The patient was alive and well without disease, at an examination conducted two years after first referral to me.

### CASE HISTORY 11

ES (male): Diagnosis and History: At age about 62 years, before referral to me, a transitional cell cancer of the bladder was removed surgically and followed by conventional x-ray therapy to the pelvis. First seen by me at age 63 years and 5 months, when he was referred with a recurrent cancer of the bladder in the base of the pelvis and multiple secondaries throughout the skeleton. Treatment of his bladder only, according to the invention, was undertaken within a few weeks of referral. Four years and five months later, and then again 3, 4, 6 and 11 months after that, further treatments according to the invention were given to the whole skeleton for the multiple metastases that had by then developed. Complete resolution of all symptoms was observed. His bone scan remained abnormal 5 months after completion of the final treatment course, his condition thereafter slowly improved and a report showed no evidence of progression of his disease. His bone scan three years and six months after the last treatment revealed healing at all sites of previous cancer.

### CASE HISTORY 12

PT (male): Diagnosis and History: Carcinoma of the prostate. Adenocarcinoma confirmed by biopsy. Referred for x-ray therapy at age 74 years and 5 months, before referral to me, but he declined to have this and he was treated by me according to the invention when first seen by me at age 74 years and 6 months. Over the subsequent 6 years and 7 months, when a further examination was conducted, his prostatic specific antigen had remained within normal limits. At that examination he exhibited no symptoms of disease, showed normal serum prostatic antigen levels and had normal micturition. There was no sign of disease when last seen 10 years after his initial therapy.

### CASE HISTORY 13

ST (male): Diagnosis and History: At age 7 years, before referral to me, the patient was losing weight, with slow deterioration in mental functions. Multiple investigations including a craniotomy and biopsy were made. Cerebrospinal fluid was examined twice yearly, showing steady increase in lymphocytes, raised protein levels and lower glucose levels. Investigations suggested a chronic inflammatory process. A variant of multiple sclerosis was eventually diagnosed and he was first seen by me at age 20 years and 11 months. Treatment according to the invention was given a few weeks after first referral to me, followed by further courses one and seven months after the first. After treatment, his general condition slowly improved, his cerebral functioning returned almost to normal and a cerebrospinal fluid showed that the lymphocytes in the fluid had disappeared, and the protein and glucose levels were normal. No obvious neurological defect was demonstrated by magnetic resonance image (MRI) of the brain and spinal cord. Probably the patient had a rare type of multiple sclerosis, which unquestionably has been beneficially influenced by the method of treatment according to the invention.

### CASE HISTORY 14

EJ (female): Diagnosis and History: Referred to me five years ago, when given a prognosis of 6 to 10 weeks life, from multiple lung secondary cancer arising from a primary osteogenic sarcoma in her lower left femur. This had been removed and amputation avoided by grafting, followed by one year of intensive cytotoxic chemotherapy. This had failed to have any beneficial effect on her disease, only severe complications of the chemotherapy. Treated by two courses according to the invention (five years and four years eight months ago). Chest x-rays and CT scan since treatment revealed no active cancer in her lungs or elsewhere in her body.

### CASE HISTORY 15

JB (female): Diagnosis and History: Slowly enlarging mass in the left calf attached to the peroneus longus muscle. At age about 32 years, before referral to me at age 38 years, the patient had broken her left hip and calf in a car accident and had developed a swollen left leg since that accident. The patient had experienced a one month history of loss of sensation in the area served by the common peroneal nerve. The tumour was partly removed. Histology was a myxoid liposarcoma and myxoid sarcoma 14 cm long, which was incompletely removed. The patient was referred to me at age 38 years, and a course of treatment was then given according to the invention. No evidence of recurrence was found in the subsequent 8 years and 4 months. About 5 years after the treatment according to the invention, a biopsy was carried out because of a suspicious change in the outline of her leg. The alteration in leg contour was due for further resolution of the oedema, and not recurrence, with no evidence of active malignancy.

### CASE HISTORY 16

JS (female): Diagnosis and History: Abdominal mesothelioma since age about 34 years, before referral to me, and the patient was suffering from systemic lupus erythematosis (SLE) with the classic facial rash and abnormalities of serum immunoglobulins. She was treated with conventional methods and there was a slow deterioration in her health due to SLE. First seen by me at age 54 years and 5 months, when she had intermittent left sided abdominal discomfort and the development of ascites in the peritoneal cavity. Investigation and biopsy confirmed the presence of a left sided pelvic mesothelioma of the peritoneum. Medication for her systemic lupus was Prednisolone™ 5 mg a day, Methotrexate™ 10 mg once or twice a week, Adalat™ 30 mg a day, Lipitor™ and Valium™ 10 mg a day together with Clomazepam™ 2 to 4 mg at night. Despite this medication, her disease was slowly advancing. She was treated according to the invention, when first seen by me, and two repeat courses of treatment were given 5 and 9 months after the initial treatment. Investigations, two months after completion of the treatment according to the invention, showed that the mesothelioma had shrunk considerably, the ascites had almost disappeared, the multiple hypodensities in the liver were now cystic and indicated that they had been sterilised from their original metastatic characteristics. Her IgM had fallen from 15.2 g/L immediately before referral to me to 11.6 g/L one year later (normal 0.53 ― 3.32). There was a complete disappearance of the classic SLE facial rash and the patient ceased taking all her medications for SLE and reported that the symptoms had all completely disappeared. Treatement for her mesothelioma was repeated twice at 3 month intervals. Reviewed three years and six months after her first treatment according to the invention, she had no clinical evidence of SLE (IgM now 5.32 ― laboratory normals lie between 0.53 and 3.32) or her peritoneal mesothelioma: a computerised tomographic (CT) x-ray scan of chest and abdomen was normal.

### CASE HISTORIES 17-22

Thoracic mesotheliomata ― the following Table I summarises the case Histories of six mesotheliomata patients who have been treated according to the invention and all survived for more than three years with static x-ray appearances for more than one year after completion of the final treatment according to the invention.

**Table I**

| Case History No: | Sex | Age at Diagnosis | Lung Site: Left or Right | Effusion Treat- ment | First Treatment (months after diagnosis) | Months Known Survival After First Treatment | Comments NSR = No sign of recurrence |
|---|---|---|---|---|---|---|---|
| 17 | F | 49 | L | Yes, Drained | 2 | 162 | Alive, NSR |
| 18 | M | 44 | L | Yes, Drained | 3 | 198 | Died, NSR |
| 19 | F | 47 | Both | Yes, Drained | 2 | 48+ | NSR* |
| 20 | M | 41 | L | Yes, Drained | 1 | 36+ | NSR, LSO |
| 21 | M | 46 | R | Yes, Drained | 2 | 98+ | All x-rays after first treatment report "scarring" |
| 22 | M | 50 | L | No | 4 | 102+ | Alive, NSR |
| LSO = Lost sight of | | | | | | | |
| The column headed "First Treatment" refers to the first treatment according to the invention. | | | | | | | |

| | | | | | | | |
|---|---|---|---|---|---|---|---|
| *This patient had complete remission for four years after her original therapy. She later died, without any evidence of active cancer, from complications of subsequent interferon/chemotherapy for which she volunteered, for research purposes not related to my therapy, and against my advice. | | | | | | | |

The above broadly describes the present invention without limitation. Variations and modifications as will be readily apparent to those skilled in this art are intended to be included in the scope of this application and subsequent patents.

## Claims

1. Use, in the following therapeutic method or in the manufacture of one or more compositions for use in the following therapeutic method, of:
(a) one or more non-toxic organic disulfide selected from:
penicillamine disulfide (I) 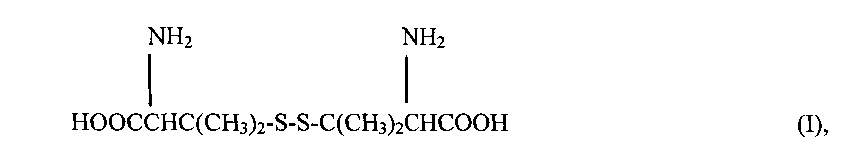
non-toxic salts thereof;
compounds of formula IV
R-S-S-R (IV)
in which R is an ethyl group β,β-disubstituted by two substituents selected from amino, carboxy, carboxy-(C₁₋₄-alkyl)-carbamoyl, C₂₋₅-alkanamido substituted by amino and carboxy,
non-toxic salts thereof;
disulfide-bridged proteins,
and non-toxic salts thereof;
and
(b) one or more non-toxic compound selected from:
cysteine sulfoxides of general formula VI 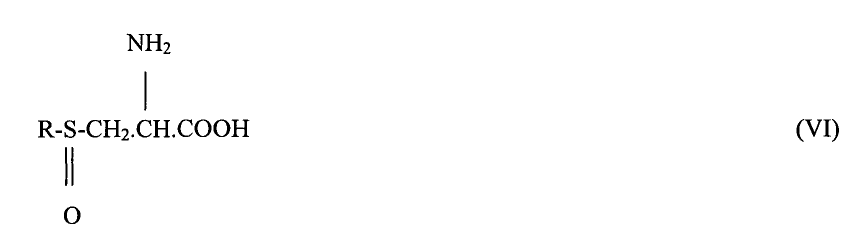
in which R is a C₁₋₄ alkyl or a C₂₋₄ alkenyl group,
non-toxic salts thereof,
compounds of general formula VII 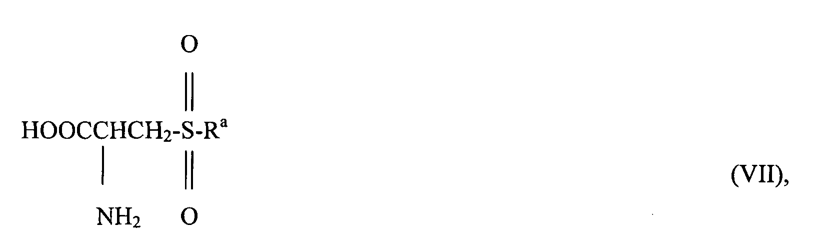
in which R^{a} is H, OH or a C₁₋₄ alkyl or a C₂₋₄ alkenyl group,
and non-toxic salts thereof;
the said therapeutic method being selected from treating cancer, enhancing T-cell count, or reducing the concentration of pathogenic particles (e.g. virus or prion particles) - most especially those selected from human immunodeficiency virus (HIV), bovine spongiform encephalopathy (BSE) agent, scrapie agent, hepatitis agent, disseminated ("multipie") sclerosis agent, Creutzfeld Jacob disease (CJD) agent, variant CJD agent, systemic lupus erythematosis (SLE) agent, ankylosing spondylitis agent and amyotrophic sclerosis agent - applicable *in vivo* to a patient in need of such treatment, the method comprising administering to the patient an effective amount of the agents (a) and (b), and, while the agents are present, administering to the patient an effective dose of electromagnetic radiation of frequency in the range of about 400-450 MHz;
with the provisos that:
for treatment of cancer, the combination of (a) cystine, oxidised glutathione, any non-toxic salt of either, or any mixture or combination thereof and (b) one or more non-toxic cysteine sulfoxide of general formula VI, any non-toxic salt thereof, or any mixture or combination thereof is excluded; and
for reducing the concentration of pathogenic particles, the combination of (a) cystine, oxidised glutathione, any non-toxic salt of either, or any mixture or combination thereof and (b) one or more non-toxic cysteine sulfoxide of general formula VI, any non-toxic salt thereof, or any mixture or combination thereof is excluded.

2. Use, in the following therapeutic method or in the manufacture of a composition for use in the following therapeutic method, of:
(a) one or more non-toxic organic disulfide selected from: penicillamine disulfide (I), non-toxic salts thereof, compounds of formula IV, non-toxic salts thereof, disulfide-bridged proteins, and non-toxic salts thereof;
(b) one or more non-toxic compound selected from: cysteine sulfoxides of general formula VI, non-toxic salts thereof, compounds of general formula VII, and non-toxic salts thereof; and
(c) one or more non-toxic compound selected from:
organic sulfoximines of general formula 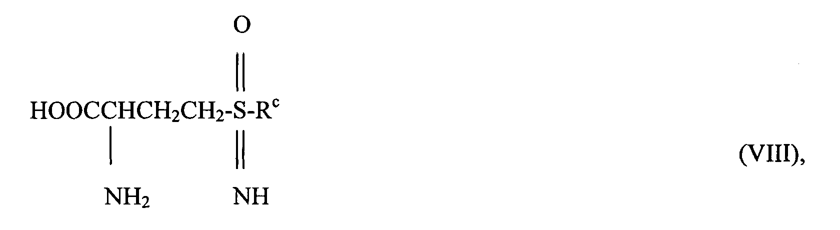 wherein R^{c} is a C₁₋₄ alkyl group, for example methyl or butyl,
and non-toxic salts thereof;
the said therapeutic method being selected from treating amyloid conditions, Alzheimer's disease, and non-malignant diseases such as SLE, multiple sclerosis and scleroderma, applicable *in vivo* to a patient in need of such treatment, the method comprising administering to the patient an effective amount of the agents (a), (b) and (c), and, while the agents are present, administering to the patient an effective dose of electromagnetic radiation of frequency in the range of about 400-450 MHz.

3. Use according to claim 1 or claim 2, wherein the compound of formula IV or a non-toxic salt thereof is selected from cystine and oxidised glutathione or a non-toxic salt thereof.

4. A method of treating cancer, of enhancing T-cell count, or of treating acquired immunodeficiency syndrome (AIDS), bovine spongiform encephalopathy (BSE), scrapie, hepatitis, disseminated ("multiple") sclerosis, Creutzfeld Jacob disease (CJD), variant CJD, systemic lupus erythematosis (SLE), ankylosing spondylitis and amyotrophic sclerosis *in vivo* in a patient in need of such treatment, the method comprising administering to the patient an effective amount of the agents (a) and (b) as defined in claim 1 or claim 3 when dependent on claim 1, and, while the agents are present in the patient administering to the patient an effective dose of electromagnetic radiation of frequency in the range of about 400-450 MHz.

5. A method of treating amyloid conditions, Alzheimer's disease, and non-malignant diseases such as SLE, multiple sclerosis and scleroderma *in vivo* in a patient in need of such treatment, the method comprising administering to the patient an effective amount of the agents (a), (b) and (c) as defined above in claim 2 or claim 3 when dependent on claim 2, and, while the agents are present in the patient administering to the patient an effective dose of electromagnetic radiation of frequency in the range of about 400-450 MHz.

6. A kit for use in a method as defined in claim 4, the kit comprising a first composition or compositions containing the (a)-agent or agents and a second composition or compositions containing the (b)-agent or agents, optionally together with instructional material for the use.

7. A kit for use in a method as defined in claim 5, the kit comprising a first composition or compositions containing the (a)-agent or agents, a second composition or compositions containing the (b)-agent or agents, and a third composition or compositions containing the (c)-agent or agents, optionally together with instructional material for the use.

8. An aqueous pharmaceutical composition comprising (a) one or more non-toxic organic disulfide selected from: penicillamine disulfide (I), non-toxic salts thereof, compounds of formula IV, non-toxic salts thereof, disulfide-bridged proteins, and non-toxic salts thereof; and (b) one or more non-toxic compound selected from: cysteine sulfoxides of general formula VI, non-toxic salts thereof, compounds of general formula VII, and non-toxic salts thereof; with the proviso that when the composition includes the specific combination of (a) cystine, oxidised glutathione, any non-toxic salt of either, or any mixture or combination thereof and (b) one or more non-toxic cysteine sulfoxide of general formula VI, any non-toxic salt thereof, or any mixture or combination thereof, then at least one further compound from those defined for (a) and (b) in claim 1 is also present.

9. A pharmaceutical composition according to claim 8, wherein the compound of formula IV or an non-toxic salt thereof is selected from cystine, oxidised glutathione or a non-toxic salt thereof.

10. Use, in the following therapeutic method or in the manufacture of one or more compositions for use in the following therapeutic method, of one or more non-toxic compound selected from: compounds of general formula VII, non-toxic salts thereof, and compounds of general formula VIIa, 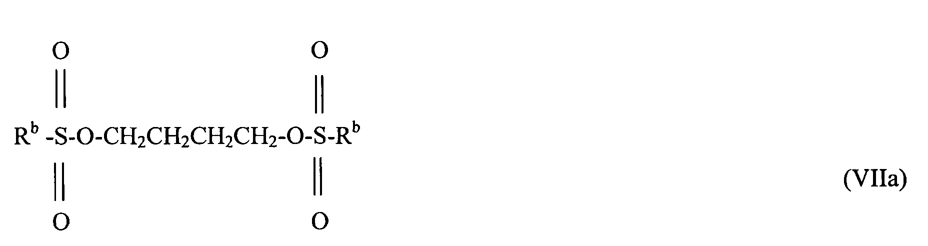 in which R^{b} is a C₁₋₄ alkyl or a C₂₋₄ alkenyl group, preferably methyl;
the said therapeutic method being selected from treating cancer, enhancing T-cell count, treating amyloid conditions, Alzheimer's disease, and non-malignant diseases such as SLE, multiple sclerosis and scleroderma or reducing the concentration of pathogenic particles (e.g. virus or prion particles) ― most especially those selected from human immunodeficiency virus (HIV), bovine spongiform encephalopathy (BSE) agent, scrapie agent, hepatitis agent, disseminated ("multiple") sclerosis agent, Creutzfeld Jacob disease (CJD) agent, variant CJD agent, systemic lupus erythematosis (SLE) agent, ankylosing spondylitis agent and amyotrophic sclerosis agent - applicable *in vivo* to a patient in need of such treatment, the method comprising administering to the patient an effective amount of the one or more compound of general formula VII or a non-toxic salt thereof, one or more compound of general formula VIIa, or any combination thereof, and, while the compound or compounds are present, administering to the patient an effective dose of electromagnetic radiation of frequency in the range of about 400-450 MHz.

11. A method of treating cancer, of enhancing T-cell count, of treating amyloid conditions, Alzheimer's disease, and non-malignant diseases such as SLE, multiple sclerosis and scleroderma or of treating acquired immunodeficiency syndrome (AIDS), bovine spongiform encephalopathy (BSE), scrapie, hepatitis, disseminated ("multiple") sclerosis, Creutzfeld Jacob disease (CJD), variant CJD, systemic lupus erythematosis (SLE), ankylosing spondylitis and amyotrophic sclerosis *in vivo* in a patient in need of such treatment, the method comprising administering to the patient an effective amount of one or more non-toxic compound selected from: compounds of general formula VII, non-toxic salts thereof, and compounds of general formula VIIa, and, while the said compound or compounds are present in the patient administering to the patient an effective dose of electromagnetic radiation of frequency in the range of about 400-450 MHz.

12. A use or method according to claim 10 or 11, wherein the one or more compound of general formula VII or a non-toxic salt thereof, one or more compound of general formula VIIa, or any combination thereof, can be administered in association with one or more of the other components (a), (b) and (c) as defined in any one of claims 1 to 3.

13. A kit for use in a method as defined in claim 10 or claim 11, the kit comprising a composition containing one or more compound selected from: compounds of general formula VII, non-toxic salts thereof, and compounds of general formula VIIa, optionally in association with one or more additional composition or compositions containing one or more of the other the (a)-agent or agents, (b)-agent or agents, and (c)-agent or agents as defined above, optionally together with instructional material for the use.

14. An aqueous pharmaceutical composition comprising one or more non-toxic compound selected from: compounds of general formula VII, non-toxic salts thereof, compounds of general formula VIIa, and co-agents selected from: (a) one or more non-toxic organic disulfide selected from: penicillamine disulfide (I), non-toxic salts thereof, compounds of formula IV, non-toxic salts thereof, disulfide-bridged proteins, and non-toxic salts thereof, (b) one or more compound selected from: cysteine sulfoxides of general formula VI and non-toxic salts thereof, and (c) one or more compound selected from: organic sulfoximines of general formula (VIII) and non-toxic salts thereof; or any combination thereof.

15. A pharmaceutical composition according to claim 14, wherein the compound of formula IV or a non-toxic salt thereof is selected from cystine, oxidised glutathione and non-toxic salts thereof.

16. A use, method or kit according to any one of claims 1 to 7 and 10 to 13, wherein the ative agent or combination of active agents consists essentially of the stated active agent or combination of agents with a pharmaceutically acceptable carrier, diluent, excipient or combination thereof.

17. A pharmaceutical composition according to any one of claims 8, 9, 14 and 15, which consists essentially of the stated active agent or combination of agents with a pharmaceutically acceptable carrier, diluent, excipient or combination thereof.
